# EUROPEAN PATENT SPECIFICATION

(11) **EP 0 697 402 B1**
(45) Date of publication and mention of the grant of the patent: **29.12.1999**
(21) Application number: 94870138.8
(22) Date of filing: 19.08.1994
(51) Int. Cl.: C07C 333/04

(54) **Manufacturing process for the production of prosulfocarb**
Verfahren zur Herstellung von Prosulfocarb
Procédé pour la préparation de prosulfocarb

(43) Date of publication of application: 21.02.1996
(73) Proprietor: NV MONSANTO EUROPE SA, 1150 Brussels (BE)
(72) Inventor: Dekeyser, Eddy F. M., B-9080 Lochristi (BE); Vermeulen, Eddy G. D., B-2091 Stabroek (BE)
(74) Representative: Colens, Alain

(56) References cited:
- DE-A- 2 844 305
- FR-A- 1 328 112

## Description

The present invention relates to a manufacturing process for the production of prosulfocarb or S-benzyl-N,N-di-n-propylthiocarbamate.

Prosulfocarb is an ester of thiolcarbamic acid. Some of these esters are well-known herbicides . Triallate (S-2,3,3-trichloroallyl diisopropyl thiocarbamate ), esprocarb (S-benzyl-N-ethyl-N-(1,2- dimethylpropyl) thiocarbamate) and prosulfocarb are representative of these esters and are commercially available herbicides of this family.

Several processes have been developed for the manufacture of thiolcarbamate esters, using either phosgene (COCl₂) or carbonoxysulfide (COS) as starting materials. According to one well-known method, the halide corresponding to the desired ester is condensed with an alkali metal thiocarbamate which is obtained by reaction of carbonoxysulfide with the corresponding amine in the presence of alkali.

In the latter case, both one-step and two-step methods have been applied. US patent 3,330,643 discloses thiolcarbamate esters with herbicidal activity, the esters being synthesised by a two-step method that is to say first reacting carbonoxysulfide with the amine and second, reacting the corresponding alkali salt with the appropriate alkyl halide.

French patent 1328112 describes also a two-step process. The intermediate is a sodium salt.

German patent DE-A-28 44 305 describes a process for producing thiolcarbamates which is again a traditional two-step process with consecutive reaction sequence. No addition of a separate aqueous base such as sodium hydroxide is described. The intermediate is an ammonium salt of thiocarbamic acid. The thiolcarbamate separates as an organic phase.

For the synthesis of triallate , a one-step process may conveniently be carried out by adding carbonoxysulfide to diisopropylamine in the presence of 1,1,2,3-tetrachloropropene. Under the appropriate conditions, the latter compound will not significantly react with the amine.

In the case of prosulfocarb, however, the alkyl halide is benzylchloride and, in a one-step process, this particular halide leads to an undesirable side-reaction with the amine to provide benzyl di-n-propylamine as a by-product. According to the known prior art, a two-step process should therefore be elected despite a longer batch time will then be needed.

There is nevertheless still a need to obtain as short a batch time as possible together with a high yield of product of high purity and low consumption of reagents.

It has now been observed that it is possible to shorten the batch time for manufacturing prosulfocarb by adding benzylchloride to the reaction mass already at a point where a substantial amount of carbonoxysulfide has reacted with the amine. Surprisingly the amount of by-product, benzyl di-n-propylamine, is then comparable or even lower to what may be achieved with a two-step process; that means that the undesired side reaction is essentially suppressed.

Preferably, the addition of benzylchloride and the addition of carbonoxysulfide are terminated simultaneously, at a time corresponding to the termination of the addition of carbonoxysulfide for the corresponding one-step process.

Furthermore it has been observed that the presence of sodium hydroxide is not mandatory at the early stage of the reaction. In the presence of the excess of amine, an ammonium thiocarbamate salt is indeed produced in lieu of the corresponding alkali salt. pH control by addition of an aqueous base, typically a sodium hydroxide solution, is needed only after the addition of benzylchloride has begun.

The invention is thus directed to a process for producing S-benzyl- N,N-di-n-propylthiocarbamate from carbonoxysulfide, N,N-di-n-propyl amine and benzylchloride wherein carbonoxysulfide is added continuously to a mixture of aqueous N,N-dipropylamine characterised in that benzylchloride is started to be added continuously to the reaction mixture at a point where approximately 30-60 % of a substantially equimolar amount of carbonoxysulfide has been added.

The invention is also directed to a process for producing S-benzyl- N,N-di-n-propylthiocarbamate from carbonoxysulfide, N,N-di-n-propyl amine and benzylchloride wherein no sodium thiocarbamate is produced at the early stage of the reaction.

Carbonoxysulfide is available as a gas in cylinders. Preferably however, in a manufacturing line, it is produced in a separate generator for direct consumption. In the generator, carbonoxysulfide is produced by the reaction of sulfuric acid with concentrated aqueous ammonium thiocyanate at 80-90°C.

The resulting carbonoxysulfide gas is then sparged into the agitated reaction vessel containing the di-n-propylamine/water mixture. According to one embodiment, the corresponding amine/carbonoxysulfide molar ratio is approximately 1.3, but more specifically 1.4 mole of amine may be used per mole of benzylchloride. The corresponding ammonium thiocarbamate, an intermediate salt, is then formed.

Preferably the temperature in the reaction vessel is maintained between 5 and 15 °C. The reactor back-pressure is preferably kept under 250 mmHg.

When a substantial portion of the COS has been charged, the benzylchloride slow addition is initiated, which starts to liberate HCl. Preferably the slow addition starts at a point where approximately 30 to 60 %, more preferably about 50%, of the COS has been charged.

For example, the addition of COS will take 100 minutes but the addition of benzylchloride is initiated after 50 minutes.

According to another aspect of the invention, an aqueous base, preferably sodium hydroxide is also slowly added, under pH control, in order to neutralise the HCl generated by the reaction of benzylchloride. 22% aqueous sodium hydroxide is typically used. Meanwhile the remainder of the COS is charged at the same rate as before and, advantageously, both slow additions are terminated simultaneously at which point substantially equimolar amounts of each have been added.

After the addition is complete, the reaction mixture is left to react for a hold time and the temperature is allowed to increase, for example from around 8°C up to 40°C. In the case of batches involving 190 g of benzylchloride the hold time is typically between 10 and 60 minutes, preferably about 30 minutes.

The mixture is then neutralised by slow addition of an aqueous acid. Preferably the acid is 96 % sulfuric acid. For the above mentioned batches such acidification step will take about 20 minutes, the rate of neutralisation being dependent on the temperature profile. The temperature is controlled such that it remains below 45°C.

The resulting two-phase mixture is separated and the organic phase containing the prosulfocarb may be purified by the usual techniques known in the art.

Typically, the yield reaches 95% of theory for the crude product (based on benzylchloride) with an assay of 97%.

Preferably purification of prosulfocarb involves a steam-stripping step under reduced pressure. This method allows one to obtain the product with an assay of more than 99%, with limited loss of yield.

The manufacturing process of the present invention including the best mode will be made clear by the following example.

### Example

Carbonoxysulfide gas was added over a period of 100 minutes (main reactor) to a stirred mixture of 209 g (2.06 moles) of di-n-propylamine (min. purity 99%) and 152 g of water at 5-15°C. Carbonoxysulfide gas was synthesised simultaneously in a second reactor vessel (generator) by slow addition of 246 g (1.62 moles) of a 50% ammonium thiocyanate aqueous solution over a period of 100 minutes, to a stirred solution containing 309 g of 96% sulfuric acid and 202 g of water at 80-90°C. The acid concentration in the latter vessel was maintained at a suitable level by slow addition of an extra 184 g of 96% sulfuric acid, starting at 19 minutes and ending at 85 minutes. The generated COS was immediately sparged into the main reactor contents. Slow addition of 190 g (1,47 moles) of benzylchloride (min purity 98 %) to the main reactor was started when 130 g (0.85 moles) of 50% ammonium thiocyanate had been added to the generator. The addition of benzylchloride was carried out over the remaining period of 45 minutes. The pH of the reaction mixture was allowed to drop from its initial value to 11, where it was maintained by continuous addition of a 22% aqueous sodium hydroxide solution. These conditions required only sodium hydroxide addition after the beginning of the benzylchloride addition. Ammonium thiocyanate addition to the generator and benzylchloride addition to the main reactor were terminated simultaneously at 100 minutes. With the exception of a sufficient venting period (typically during addition of the first 20 g of ammonium thiocyanate), the vent of the main reactor to the atmosphere was kept closed. The back-pressure in the main reactor did not exceed 3.07 10⁴ Pa (230 mmHg).

The back-pressure in the system was released and nitrogen gas was sparged through the contents of the generator during 5 minutes, in order to transfer residual carbonoxysulfide from the generator to the main reactor.

The mixture in the main reactor was stirred during an additional 30 minutes, while heated to approximately 40°C. During completion of the reaction, sodium hydroxide was further added to the mixture under pH control. At the end of the hold period an approximately stoichiometric total amount of sodium hydroxide (approx. 1.47 moles) had been added to the mixture.

96% sulfuric acid was then slowly added to the mixture under stirring, while keeping the temperature below 45°C, until the pH reached 2. The mixture was further kept under stirring for an additional 10 minutes.

The pH of the mixture was then brought back to 5.8 by slow addition of 22 % aqueous sodium hydroxide under stirring, whilst not exceeding 45°C.

The top organic layer was separated and analysed by capillary gas chromatography. A 95% yield of S-benzyl-N,N-di-n-propylthiocarbamate with an assay of 97 % was obtained.

Steam stripping of the organic layer at 120-130 °C and 50 mbar resulted in a 94 % overall yield of S-benzyl-N,N-di-n-propyl thiocarbamate with 99 % purity, as analysed by capillary gas chromatography.

The generator vessel was a jacketed 1.2 litre glass reactor equipped with an agitator, glass frit (sparger), heating unit, and pressure and temperature measurement devices.

The main reactor was an identical jacketed 1.2 litre glass reactor equipped with an agitator, glass frit (sparger) heating/cooling unit, pressure and temperature measurement devices, and a pH measurement/control device.

Prosulfocarb is a clear liquid soluble in acetone, chlorobenzene, ethanol, kerosene and xylene. It may be dispersed directly in water or a solution thereof in an appropriate organic solvent may be emulsified in aqueous medium by the aid of a dispersing agent known in the art. In general, admixture with a small amount of an organic surface active agent capable of lowering the surface tension of water is desirable for field applications. Suitable surface active agents may easily be selected by the person skilled in the art. Dry formulations for application as dusts may be accomplished by mixing the active material with a finely divided carrier known per se. Absorption of the active material on inert granules may also provide effective forms for field applications. Prosulfocarb may be applied pre-emergent or early post-emergent to winter wheat, winter barley and rye, to control grasses and broadleaf weeds, especially Galium aparine, Alopecurus myosuroides, Lolium multiflorum, Poa annua, Sinapis arvensis, Stellaria media and Veronica spp.

## Claims

1. A manufacturing process for the production of S-benzyl-N,N-di-n-propylthiocarbamate from carbonoxysulfide, N,N-di-n-propyl amine and benzylchloride wherein carbonoxysulfide is added continuously to a mixture of aqueous N,N-di-n-propylamine characterised in that benzylchloride is started to be added continuously to the reaction mixture only at a point where approximately 30-60 % of the total quantity of carbonoxysulfide has been added, the addition of benzylchloride being performed under pH control by addition of an aqueous base.

2. The process according to Claim 1 wherein the addition of the benzylchloride is performed under pH control with sodium hydroxide.

3. The process according to Claim 2 wherein the pH control is performed by adding a NaOH solution in a fixed ratio with benzylchloride until a stoichiometric amount of NaOH has been added.

4. The process of Claim 1 wherein the addition of carbonoxysulfide and the addition of benzylchloride are terminated simultaneously.

5. The process according to any preceding claims wherein an excess of amine is used.

6. The process according to Claim 5 wherein 1.4 mole of amine is used per mole of benzylchloride.

7. The process according to any preceding claims wherein the pH value is maintained between 10.5 and 13.0.

## Patentansprüche

1. Herstellungsverfahren für die Erzeugung von S-Benzyl-N,N-di-n-propylthiocarbamat aus Carbonoxysulfid, N,N-Di-n-propylamin und Benzylchlorid, wobei Carbonoxysulfid kontinuierlich zu einer Mischung aus wäßrigem N,N-Di-n-propylamin gegeben wird, dadurch gekennzeichnet, daß die kontinuierliche Zugabe von Benzylchlorid zu der Reaktionsmischung erst an einem Punkt begonnen wird, bei dem etwa 30-60% der Gesamtmenge an Carbonoxysulfid zugegeben worden sind, wobei die Zugabe von Benzylchlorid unter pH-Regulierung durch Zugabe einer wäßrigen Base durchgeführt wird.

2. Verfahren nach Anspruch 1, wobei die Zugabe des Benzylchlorids unter pH-Regulierung mit Natriumhydroxid durchgeführt wird.

3. Verfahren nach Anspruch 2, wobei die pH-Regulierung durch Zugabe einer NaOH-Lösung in einem festgelegten Verhältnis zu Benzylchlorid durchgeführt wird, bis eine stöchiometrische Menge an NaOH zugesetzt worden ist.

4. Verfahren nach Anspruch 1, wobei die Zugabe von Carbonoxysulfid und die Zugabe von Benzylchlorid gleichzeitig beendet werden.

5. Verfahren nach mindestens einem der vorangehenden Ansprüche, wobei ein Überschuß an Amin verwendet wird.

6. Verfahren nach Anspruch 5, wobei 1,4 Mol Amin pro Mol Benzylchlorid verwendet werden.

7. Verfahren nach mindestens einem der vorangehenden Ansprüche, wobei der pH-Wert zwischen 10,5 und 13,0 gehalten wird.

## Revendications

1. Procédé de fabrication destiné à la production de S-benzyl-N,N-di-n-propylthiocarbamate à partir d'oxysulfure de carbone, de N,N-di-n-propylamine et de chlorure de benzyle dans lequel l'oxysulfure de carbone est ajouté en continu à un mélange de N,N-di-n-propylamine aqueux, caractérisé en ce que l'on commence à ajouter le chlorure de benzyle en continu au mélange réactionnel uniquement à un instant où approximativement 30 à 60 % de la quantité totale d'oxysulfure de carbone ont été ajoutés, l'addition de chlorure de benzyle étant réalisée sous conmande du pH par l'addition d'une base aqueuse.

2. Procédé selon la revendication 1, dans lequel l'addition du chlorure de benzyle est réalisée sous commande du pH par de l'hydroxyde de sodium.

3. Procédé selon la revendication 2, dans lequel la commande du pH est réalisée en ajoutant une solution de NaOH selon un rapport fixe avec le chlorure de benzyle jusqu'a ce qu'une quantité stoechiométrique de NaOH ait été ajoutée.

4. Procédé selon la revendication 1, dans lequel l'addition d'oxysulfure de carbone et l'addition de chlorure de benzyle sont achevées simultanément.

5. Procédé selon l'une quelconque des revendications précédentes, dans lequel un excès d'amine est utilisé.

6. Procédé selon la revendication 5, dans lequel 1,4 mole d'amine est utilisée par mole de chlorure de benzyle.

7. Procédé selon l'une quelconque des revendications précédentes, dans lequel la valeur de pH est maintenue entre 10,5 et 13,0.
